# EUROPÄISCHE PATENTANMELDUNG

(11) **EP 4 707 377 A2**
(43) Veröffentlichungstag der Anmeldung: **11.03.2026**
(21) Anmeldenummer: 25193380.0
(22) Anmeldetag: 01.08.2025
(51) Int. Cl.: C12N 1/12

(54) **NÄHRMITTELSYSTEM FÜR BIOLOGISCHE ORGANISMEN ODER ZELLSYSTEME**

(30) Priorität: 09.08.2024 DE 102024002592
(71) Anmelder: Hochschule Kaiserslautern, Körperschaft des öffentlichen Rechts, 67659 Kaiserslautern (DE)
(72) Erfinder: Lakatos, Michael, 67663 Kaiserslautern (DE); Wolf, BSc, Julian, 66953 Pirmasens (DE); Zabicki, MSc, Marek Daniel, 66953 Pirmasens (DE)
(74) Vertreter: Patentanwaltskanzlei Vièl & Wieske PartGmbB

(57) **Zusammenfassung**

Diese Erfindung betrifft ein Nährmittelsystem für biologische Organismen oder Zellsysteme, wobei mit dem Nährmitttelsystem ein Nährmedium ansetzbar bzw. regenerierbar ist, indem einer Flüssigkeit, die ein Nährmedium bilden soll, oder einem bestehenden Nährmedium ein verschiedene Nährstoffe enthaltendes Nährmittel hinzugefügt wird, wobei das Nährmittel miteinander unverträgliche Nährstoffe enthält, die die Haltbarkeit des Nährmittels im Lagerzustand verringern, wenn Sie miteinander reagieren, wobei das Nährmittelsystem wenigstens zwei Nährmittelkomponenten umfasst, wobei wenigstens eine Nährmittelkomponente verschiedene miteinander verträgliche Nährstoffe enthält, und wobei zumindest zwei miteinander unverträgliche Nährstoffe in verschiedene Nährmittelkomponenten eingebracht werden. Weiter betrifft die Erfindung ein Verfahren zum Herstellen oder Regenerieren eines Nährmediums.

## Beschreibung

Diese Erfindung betrifft ein Nährmittelsystem für biologische Organismen oder Zellsysteme und ein Verfahren zum Herstellen oder Regenerieren eines Nährmittels mittels eines Nährmittelsystems.

Unter einem Nährmittelsystem wird ein Nährmittel für biologische Organismen oder Systeme, insbesondere Mikroorganismen und Zellkulturen, verstanden, das im Lagerzustand mehrere, voneinander getrennte Nährmittelkomponenten aufweist.

Ein Nährmedium umfasst eine Menge von Flüssigkeit sowie darin gelöste Nährstoffe. Es wird verwendet, um damit biologische Kulturen zu ernähren. Für viele bekannte Nährmedien sind optimale Konzentrationen der enthaltenen Nährstoffe bekannt. Diese werden üblicherweise beim Herstellen eines Nährmediums herbeigeführt. Durch den Verbrauch von Nährstoffen verringert sich deren Konzentration wieder. Die Ernährungsleistung des Nährmediums wird durch die verringerten oder veränderten Konzentrationen herabgesetzt. Es ist möglich, Nährstoffe zuzusetzen, um deren Konzentration wieder der optimalen Konzentration anzunähern. Auf diese Weise kann ein Nährmedium regeneriert werden. Wenn eine bestimmte Nährstoffkonzentration erreicht werden soll, hängt die zuzusetzende Menge des Nährstoffs auch von der Menge des Nährmediums ab.

Im Stand der Technik ist es bekannt, ein Nährmedium anzusetzen, indem mehrere Flüssigmedien, die jeweils Nährstoffe enthalten, mit Wasser verdünnt werden. Üblicherweise wird sogenannte Stammlösung verwendet, die zum Verdünnen vorgesehen ist. Sie kann auch als Konzentrat eines Nährstoffs angesehen werden. Das Pipettieren ist jedoch aufwendig und daher kostenintensiv.

Es ist auch bekannt, zum Ansetzen eines Nährmediums ein einziges flüssiges vorgemischtes und konzentriertes Nährmittel zu verwenden, das verschiedene Nährstoffe enthält. Dieses kann für den Gebrauch verdünnt werden. Dadurch wird der Aufwand des Dosierens und Anmischens der Komponenten eingespart. Nachteilig ist jedoch, dass dieses Nährmittelkonzentrat gekühlt werden muss und dennoch eine Haltbarkeit von nur wenigen Wochen aufweist.

Es ist auch bekannt, ein oder mehrere Nährstoffpulver in Wasser aufzulösen, um ein Nährmedium anzusetzen. Beispielsweise gibt es in einer vorgegebenen Zusammensetzung vorgemischtes Pulver für das Nährmedium BG11 nach Rippka und Herdman, das als Nährmedium zum Beispiel für Mikroalgen verwendet wird und weithin bekannt ist. Nachteilig ist daran jedoch, dass das Pulver keine hohe Stabilität aufweist, da in derartigen Mischungen zahlreiche zum Teil stark hygroskopische Inhaltsstoffe vorkommen, die eine Dosierung mittels Einwaage sehr ungenau machen. Des Weiteren besteht eine solche Pulvermischung aus zahlreichen Substanzen unterschiedlicher Dichte, was eine Entmischung stark begünstigt und zu Fehldosierungen führt. Weiterhin ist zur Dosierung des Pulvers eine Waage erforderlich.

Nachteilig an einer Mischung der bekannten Nährstoffe zur Herstellung eines Nährmediums, beispielsweise eines BG11-Mediums, ist die kurze Haltbarkeit einer solchen Mischung. Dies hat zur Folge, dass entsprechend kurzfristige Lagerhaltung für alle Nährstoffe geplant werden muss und Nährmedium vor Gebrauch frisch und arbeitsintensiv angesetzt werden muss. Zudem lässt sich vorab nicht immer feststellen, ob ein Nährstoff aufgrund von Alterung noch geeignet ist, so dass ein untaugliches Nährmedium unter Umständen erst nach seiner Fertigstellung als solches erkannt wird, wodurch der Herstellaufwand und die eingesetzten Stoffe verloren sind.

Aufgabe der Erfindung ist es, ein Nährmittelsystem zur Herstellung oder zur Regenerierung eines Nährmittels im Lagerzustand zu verbessern, das eine gute Haltbarkeit aufweist und leicht zu handhaben ist.

Gegenstand der Erfindung ist ein Nährmittelsystem für biologische Organismen oder Zellsysteme gemäß Anspruch 1.

Zudem wird ein Verfahren zum Herstellen oder Regenerieren eines verschiedene Nährstoffe enthaltenden Nährmediums durch das Nährmittelsystem vorgeschlagen. Mit dem Verfahren kann ein Nährmedium durch Hinzufügen eines Nährmittels in Form eines Nährmittelsystems gemäß Anspruch 1 zu einer Flüssigkeit oder zu einem bestehenden Nährmedium hergestellt oder regeneriert werden.

Das Nährmittel enthält mindestens zwei miteinander unverträgliche Nährstoffe, die die Haltbarkeit des Nährmittels verschlechtern, wenn Sie miteinander reagieren, wie dies beispielsweise der Fall bei Natriumcarbonat und Zitronensäure im BG11-Medium ist. Das Nährmittelsystem, mit dem das Nährmittel hergestellt oder regeneriert werden kann, enthält wenigstens zwei Nährmittelkomponenten. Wenigstens eine Nährmittelkomponente enthält mehrere verschiedene Nährstoffe. Zumindest zwei miteinander unverträgliche Nährstoffe sind in verschiedene Nährmittelkomponenten eingebracht.

Die Unteransprüche betreffen bevorzugte Weiterbildungen der Erfindung.

Die Nährmittelkomponenten können aus mehreren verschiedenen Nährstoffen zusammengesetzt sein.

Vorzugsweise weisen die Nährmittelkomponenten unterschiedliche Zusammensetzung der enthaltenen Nährstoffe auf. Denkbar ist, dass verschiedene Nährmittelkomponenten einen oder mehrere gleiche Nährstoffe enthalten. Weiter wird vorgeschlagen, dass wenigstens eine andere Nährmittelkomponente nur einen Nährstoff enthält, beispielsweise wenn ein bestimmter Nährstoff wie z.B. N, P oder NaCl in unterschiedlichen Konzentrationen Verwendung finden soll, oder der pH mit pH-beeinflussenden Stoffen auf eine bestimmte Konzentration eingestellt werden soll.

Es werden vorzugsweise zumindest zwei miteinander verträgliche Nährstoffe in einer Nährmittelkomponente zusammengefasst. Bevorzugt werden mehrere, besonders bevorzugt alle miteinander verträglichen Nährstoffe in einer Nährmittelkomponente zusammengefasst. Auf diese Weise kann die Anzahl der Nährmittelkomponenten minimiert werden.

In einer Ausführungsform enthält wenigstens eine Nährmittelkomponente mehrere Nährstoffe. Ein Vorteil davon liegt darin, dass die Zahl der in eine Flüssigkeit oder ein Nährmedium einzubringenden Nährmittelkomponenten reduziert wird. Dadurch kann auch die Lagerhaltung sowie Aufwendungen beim Einkauf und Transport reduziert werden. Dies wiederum verringert den Aufwand bei der Anwendung und unterscheidet die Erfindung vom schlichten Zusammenmischen der einzelnen Nährstoffe.

Vor dem Einbringen der Nährmittelkomponenten in die Flüssigkeit oder die Nährlösung liegen die Nährmittelkomponenten bevorzugt vollständig voneinander getrennt oder im Wesentlichen voneinander getrennt vor. Dies bedeutet, dass die Nährmittelkomponenten nicht durchmischt sind, gegebenenfalls jedoch aneinander angrenzen können. Dies könnte beispielsweise bei einer Tablette oder einem kristallinen oder polykristallinen Block der Fall sein, die bzw. der mehrere Nährmittelkomponenten umfasst. Die Nährmittelkomponenten können dabei in unterschiedlichen Abschnitten der Tablette oder der Blocks angeordnet sein. Es ist denkbar, zwischen Nährmittelkomponenten in einer Tablette oder in einem Block eine mit den Inhaltsstoffen der Nährmittelkomponenten nicht reaktive Zwischenschicht einzubringen. Auf diese Weise kann verhindert werden, dass Nährmittelkomponenten in unterschiedlichen Abschnitten in der Grenzschicht miteinander reagieren. Es ist auch denkbar, eine begrenzte Reaktion zuzulassen und die Reaktionsprodukte eine solche Zwischenschicht bilden zu lassen.

Bevorzugt bleiben vor dem Hinzufügen zu der Flüssigkeit oder zu dem bestehenden Nährmedium mit im Wesentlichen oder vollständig voneinander getrennt.

Alternativ zum oben genannten Vorschlag können die Nährmittelkomponenten im Lagerzustand im Wesentlichen oder vollständig voneinander getrennt sein. Dies bedeutet, dass die Nährmittelkomponenten nicht intensiv miteinander vermischt sind, was in erheblichem Ausmaß zu Reaktionen zwischen Nährmittelbestandteilen führen würde. Beispielsweise können die Nährmittelkomponenten als Pulver zu einem Feststoff wie etwa einer Tablette gepresst sein, wobei sich die Feststoffe nicht berühren.

Denkbar ist, dass alle Nährstoffkomponenten in einer einzigen Tablette oder einem einzigen Block angeordnet sind, wobei zwei oder mehr Nährstoffkomponenten ist der Tablette oder dem Block aneinander angrenzen. Es sind auch Kapseln für Pulver oder Gelkapseln mit mehreren Abschnitten denkbar, die jeweils mit einer Nährstoffkomponente gefüllt sind.

Es ist bereits ein Vorteil, wenn die Nährstoffe in einer Nährmittelkomponente in trockenem Zustand aneinander angrenzen. Vorteilhaft ist in trockenem Zustand die Reaktionsfähigkeit der Nährstoffe mit anderen Stoffen im Vergleich zum Vorliegen in einer Lösung reduziert. Daher hat auch eine trockne Mischung von miteinander unverträglichen Nährstoffen in einer Nährmittelkomponente die im nächsten Absatz genannten Vorteile. Gleiches wird für zwei Nährstoffe in einer Nährmittelkomponente vorgeschlagen. Auf diese Weise können weniger verträgliche in dieselbe Nährmittelkomponente eingebracht werden.

Ein Vorteil eines solchen Nährmittels ist es, dass die Haltbarkeit erheblich verlängert ist. Insbesondere kann das Nährmittelsystem im Vergleich zu einem fertig vorgemischten Nährmittel mit gleichen Bestandteilen bei höheren Temperaturen gelagert werden, um dieselbe Haltbarkeit zu erreichen.

Beispielsweise können die Nährstoffkomponenten ungekühlt bzw. bei einer Umgebungstemperatur am Lagerort, wie etwa Raumtemperatur, gelagert werden. Die Anwendung und/oder Lagerhaltung ist im Vergleich zum üblichen Vorhalten von Pulvern mit den einzelnen Nährstoffen oder in sich reaktiven flüssigen Nährstofflösungen erheblich vereinfacht. Vorteilhaft können durch Vorportionierung zum Beispiel Vorgänge wie Portionieren und/oder Auswiegen entfallen, was Personal- und Kostenaufwand einspart.

Vorzugsweise liegt wenigstens eine Nährmittelkomponente in dem Nährmittelsystem portioniert vor. Dazu kann sie zu einer Tablette gepresst oder als kristalliner oder polykristalliner Block ausgebildet sein, beispielsweise als poröser Block, wie etwa in der Art eines Zuckerwürfels. Optional können die Nährstoffe mehrerer Nährmittelkomponenten in verschiedenen Abschnitten einer Tablette oder des Blocks angeordnet sein. Dies hat den Vorteil, dass die Anwendung erleichtert und sicherer wird, indem dem Nährmedium weniger Nährmittelkomponenten hinzugefügt werden müssen. Außerdem müssen weniger Nährmittelkomponenten verpackt werden.

Alternativ ist es auch möglich, eine Nährmittelkomponente als eine Pulverportion oder als eine definierte Menge von flüssiger Lösung, insbesondere als Konzentrat, in einem Behälter zu verpacken. Die flüssige Lösung kann auch als Gel vorliegen. Die Verpackung kann z.B. als Gelkapsel ausgeführt sein. Letzteres ist grundsätzlich auch für Pulver denkbar.

Die Anwendung wird erleichtert, indem einer Flüssigkeit oder einem bestehenden Nährmedium Portionen mit definierter Nährstoffmenge zugegeben werden können, wobei zugleich füreinander schädliche Nährstoffe bei der Lagerung voneinander getrennt bleiben, wodurch die Haltbarkeit erhöht wird.

Vorzugsweise wird die Portionierung und die Menge an Nährstoffen in einer Portion so gewählt, dass das Hinzufügen der Nährmittelkomponenten zu einer definierten Menge von Flüssigkeit oder bestehendem Nährmedium, die zum Beispiel einem gängigen Volumenmaß, wie etwa einem Liter Nährmedium oder einer typischen Füllmenge eines Bioreaktors, entsprechen kann, zu einer bekannten Konzentrationserhöhung der Nährstoffe führt. Dies vereinfacht die Entscheidung über die Anzahl der hinzuzufügenden Portionen der einzelnen Nährmittelkomponenten. Bevorzugt sind die Nährstoffmengen in den Nährstoffkomponenten eines Nährmittelsystems für einen bestimmten Bioreaktor so bemessen, dass das Hinzufügen einer Portion von jeder Nährmittelkomponente zur Regeneration des Nährmediums in dem Bioreaktor führt, dessen Nährleistung nach einer typischen Gebrauchszeit signifikant nachgelassen hat. Vorzugsweise wird nach diesem Hinzufügen zumindest näherungsweise eine optimale Nährstoffkonzentration erreicht. Diese optimale Konzentration kann auf maximale Erzeugung von Biomasse ausgelegt sein. Alternativ ist jedoch auch denkbar, eine definierte Nährstoffmangelsituation zu erzeugen unter der von den Organismen ein oder mehrere bestimmte Stoffe erzeugt oder vermehrt erzeugt werden.

Um die Lagerfähigkeit zu verbessern, wird vorgeschlagen, zumindest eine der Nährmittelkomponenten des Nährmittelsystems derart zu verpacken, dass die Aufnahme von Wasser aus der Luft erschwert oder verhindert ist.

In Tabletten kann mikrokristalline Zellulose als Füllstoff eingesetzt werden. Auf diese Weise wird die Herstellung von Tabletten erleichtert. Ein Vorteil von mikrokristalliner Zellulose ist, dass sie in vielen Fällen das Nährmedium nicht stört sowie aufgrund ihrer vollständigen Unlöslichkeit in Wasser vor der Zugabe des Konzentrates der Nährmittelkomponenten zur wässrigen Lösung vollständig abgefiltert werden kann.

Das erfindungsgemäße Nährmittelsystem kann weitere Hilfsstoffe enthalten, insbesondere Magnesiumstearat und/oder Siliziumdioxid, vorzugsweise hochdisperses Siliziumdioxid.

In einer weiteren Ausführungsform sind Portionsgrößen und Zusammensetzungen der Nährmittelkomponenten derart aufeinander abgestimmt, dass eine ganzzahlige Kombination von Portionen von Nährmittelkomponenten die Zusammensetzung eines Nährmediums ergibt, insbesondere ein Standard-Nährmedium nach einem einschlägigen Rezept, beispielsweise das Nährmedium BG11.

Dies vereinfacht die Herstellung bzw. Regenerierung einer Nährlösung.

In einer weiteren Ausführungsform kann ein Nährstoff oder ein Satz von Nährstoffen als ein Basisnährmittel dienen. Das Basisnährmittel kann als eine oder mehrere Nährstoffkomponenten realisiert sein. Mit dem Basisnährmittel kann eine Grundfunktion des Nährmittels realisiert werden, beispielsweise können die Grundbedürfnisse der Mikroorganismen gedeckt werden, sodass diese überleben können oder es kann ein bestimmte Nährstoffmangelzustand herbeigeführt werden. Zusätzlich zu dem Basisnährmittel kann das Nährmittelsystem Zusatznährmittel aufweisen. Diese Zusatznährmittel können eingesetzt werden, um bestimmte Verhaltensweisen Organismen auszulösen, beispielsweise verstärktes Wachstum oder die Ausbildung bestimmter Zustände und/oder Stoffe im Stoffwechsel der Organismen. Das Basisnährmittel kann eine oder mehrere Nährmittelkomponenten umfassen, insbesondere je nach dem, inwieweit es verträgliche und unverträgliche Stoffe enthält. Es ist denkbar, das Basisnährmittel als eine oder mehrere Basistabletten oder als ein oder mehr Basisblöcke oder als Kapsel oder andere Verpackung für Flüssigkeit oder Pulver mit einer oder mehreren Abteilungen zu realisieren. Ein Zusatznährmittel kann eine oder mehrere Nährmittelkomponenten umfassen, insbesondere je nach dem, inwieweit es verträgliche und unverträgliche Stoffe enthält. Es ist möglich, dass ein Zusatznährmittel nur einen einzigen Nährstoff enthält. In Bezug auf Zusatznährmittel wird unter einem Nährstoff auch ein Stoff verstanden, der nicht zur Ernährung durch Verstoffwechselung vorgesehen ist, sondern den Stoffwechsel beeinflusst. Vorzugsweise sind die Zusatznährstoffe in anderen Nährmittelkomponenten enthalten sind als die Nährmittelkomponenten, die Basisnährstoffe enthalten. Auf diese Weise ist es möglich, durch Hinzufügen von Nährmittelkomponenten die Konzentration eines oder mehrere Zusatznährstoffe in einem Nährmedium unabhängig von der Konzentration des Basisnährstoffs oder der Basisnährstoffe einzustellen. Insbesondere kann ein Zusatznährstoff in einer Nährmittelkomponente enthalten sein, die keine anderen Nährstoffe enthält. Auf diese Weise kann die Konzentration dieses Zusatznährstoffs unabhängig von allen anderen Nährstoffen eingestellt werden.

In vielen Bioreaktoren existieren erwünschte und unerwünschte Organismen in demselben Nährmedium. Mit dem Nährmittelsystem kann ein Nährmedium als ein Mangelmedium hergestellt werden. Dies bedeutet, dass dieses Nährmedium dann eine Nährstoffmischung aufweist, die das Wachstum der erwünschten Organismen fördert und das der unerwünschten Organismen weniger stark fördert oder behindert. Neben dieser Nährstoffmischung können auch weitere Substanzen, wie zum Beispiel Antibiotika oder Fungizide, vorhanden sein. Es wird vorgeschlagen, zur Herstellung eines Mangelmediums in den Nährstoffkomponenten eine hohe Reinheit der Nährstoffe vorzusehen. Auf diese Weise werden unerwünschte Organismen weniger stark von Beiprodukten der Nährstoffe und anderen Verunreinigungen ernährt. Es gibt jedoch Nährstoffe, beispielsweise stickstoffhaltige Substanzen, die in einigen Anwendungsfällen dazu geeignet sind, sowohl die erwünschten als auch die unerwünschten Organismen zu ernähren. Optional können solche Nährstoffe als Zusatznährmittel in das Nährmittelsystem eingebunden werden.

Die Nährstoffe in dem Nährmittelsystem können mit den entsprechenden Toleranzen für Beiprodukte und/oder Verunreinigungen, insbesondere für besonders wachstumsfördernde Stoffe, etwa stickstoffhaltige oder phosphorhaltige, sowie pH-beeinflussende Stoffe, in wenigstens eine, vorzugsweise alle Nährmittelkomponenten eingebracht werden. Auf diese Weise kann ein Mangelmedium hergestellt werden. Außerdem können die Mengen der Nährstoffe mit den entsprechenden Toleranzen in die Nährstoffkomponenten eingebracht werden. Dies stellt bei niedrigen Konzentrationen der Nährstoffe, zum Beispiel zum Herbeiführen eines bestimmten Stoffwechselzustands der Organismen, sicher, dass keine unwirksamen oder schädlichen Konzentrationen auftreten.

In einer weiteren Ausführungsform wird die Portionierung einer Nährmittelkomponente derart klein gewählt, dass zumindest eine Konzentration eines Zusatznährstoffs in dem Nährmedium durch die Anzahl der Portionen dieser Nährmittelkomponente einstellbar ist. Insbesondere kann auf diese Weise beispielsweise der Stickstoff-, Phosphor- oder Salzgehalt sowie die pH-Konzentration eingestellt werden.

Vorzugsweise werden für die Einstellung der genannten Eigenschaft wenigstens zwei Stufen vorgesehen. Dabei ergibt bevorzugt eine Stufe eine Normalkonzentration der Stoffe in der Nährmittelkomponente unter der Voraussetzung, dass die Menge der Basisnährstoffe an die Menge des Mediums angepasst ist. Unter einer Normalkonzentration wird eine Konzentration eines Nährstoffs in einem Nährmedium verstanden, die für als optimale Konzentration für maximale Zunahme der Biomasse angesehen wird.

Die Portionierung dieser Nährmittelkomponente wird vorzugsweise so gewählt, dass bei der Herstellung eines Nährmediums weniger als die Hälfte einer Normalkonzentration erreicht wird. Bevorzugt kann auf diese Weise ein Mangelmedium in Bezug auf einen Nährstoff, der sowohl erwünschte als auch unerwünschte Organismen ernährt, insbesondere Stickstoff, der in stickstoffhaltigen Nährstoffen enthalten ist, hergestellt werden. In dem Mangelmedium ist insbesondere weniger Stickstoff oder weniger Phosphor als bei einer Normalkonzentration desselben Nährmediums, insbesondere das Nährmedium BG11, vorhanden.

In einer weiteren Ausführungsform umfasst eine Nährmittelkomponente wenigstens zwei Salze. Insbesondere sind die Salze Nährstoffe. Die zu den Anionen der Salze gehörigen Säuren weichen in ihrer Säurestärke bevorzugt nicht mehr als um einen akzeptablen Salz-Salz-Säurestärkefaktor voneinander ab.

Zur Berechnung des Salz-Salz-Säurestärkefaktors wird die Säurestärke der stärkeren Säure durch die Säurestärke der schwächeren Säure geteilt.

Eine solche Verteilung von Salzen des Nährmittelsystems auf wenigstens eine Nährmittelkomponente verhindert, dass eine stark störende Verdrängungsreaktion zwischen zwei Salzen stattfindet, wie sie bei zu stark abweichender Salz-Salz-Säurestärke der Säuren ihrer Anionen stattfinden würde. Häufig sind die Produkte solcher Reaktionen schlecht löslich, so dass der Nährlösung Ionen entzogen werden und ausfallende Salze als Schlamm anfallen. Ein Unterschied der Säurestärken innerhalb akzeptabler Grenzen, was zu einem akzeptablen Salz-Salz-Säurestärkefaktor analog ist, bedeutet, dass die Salze in derselben Nährmittelkomponente derart langsamere Reaktionsgeschwindigkeit miteinander aufweisen, dass von einer akzeptablen Verträglichkeit der Salze ausgegangen werden kann. Beim Vorliegen einer Nährstoffkomponente als trockenes Pulver, welches auch zu Tabletten gepresst sein kann oder als kristallines oder polykristallines Blockmaterial vorliegen kann, ist die Reaktionsgeschwindigkeit gegenüber feuchtem Pulver, einem Gel oder einer wässrigen Lösung erheblich herabgesetzt. Bei Vorliegen der Nährstoffkomponente als feuchtes Pulver oder wässrige Lösung kann der akzeptable Salz-Salz-Säurestärkefaktor daher geringer ausfallen als für Feststoffe und insbesondere trockene Feststoffe.

Vorzugsweise sind in einer Nährmittelkomponente keine Salze enthalten, deren Säurestärken der Anionen mehr als um den akzeptablen Salz-Salz-Säurestärkefaktor voneinander abweichen.

Insbesondere können ein Chlorid und ein Nitrat in derselben Nährstoffkomponente vorhanden sein, bevorzugt als Kalziumchlorid und Natriumnitrat.

In einer weiteren Ausführungsform umfasst eine Nährmittelkomponente wenigstens ein Salz und wenigstens eine Säure, wobei die Säure eine geringere oder gleiche Säurestärke wie die Säure, die zu dem Anion des Salzes gehört, aufweist, oder alternativ eine größere Säurestärke als die Säure, die zu dem Anion des Salzes gehört, aufweist und einen akzeptablen Säure-Salz-Säurestärkefaktor aufweist. Zur Berechnung des Säure-Salz-Säurestärkefaktors wird die Säurestärke der Säure durch die Säurestärke der Säure des Anions des Salzes geteilt.

Wenn der akzeptable Säure-Salz-Säurestärkefaktor gleich der Zahl 1 gewählt wird, verdrängt die schwächere Säure die Kationen nicht aus dem Salz. Es ist jedoch denkbar, eine Zahl größer als 1 zu wählen, wenn diese klein genug ist, um die Reaktion zwischen der Säure und dem Salz in akzeptablem Rahmen zu halten.

Vorzugsweise sind in einer Nährmittelkomponente keine Säuren und Salze enthalten, deren Säurestärken und deren Säurestärken der Anionen um mehr als um den akzeptablen Säure-Salz-Säurestärkefaktor voneinander abweichen.

In einer weiteren Ausführungsform weist das Nährmittelsystem wenigstens eine Nährmittelkomponente auf, welche ein Salz und wenigstens eine Säure mit einer geringeren Säurestärke als die Säurestärke der Säure des Anions des Salzes enthält. Die Kombination aus einer Säure und einem Salz in einer Nährmittelkomponente bietet den Vorteil, dass darin ein Salz mit einer starken Säure des Anions untergebracht werden kann, weil die Säure im Gegensatz zu einem Salz nicht von ihrem Kation verdrängt wird. Die Dissoziation eines Protons aus der Säure führt üblicherweise nicht zum Ausfällen von Reaktionsprodukten. Vorzugsweise weist das Nährmittelsystem zusätzlich wenigstens eine Nährmittelkomponente auf welche wenigstens zwei Salze aufweist. In der Nährmittelkomponente, in der wenigstens eine Säure vorliegt, kann ein relativ großes Spektrum von Säurestärken verschiedener Säuren vorliegen. Salze, für die ein geringerer akzeptabler Salz-Satz-Säurestärkefaktor gilt und die sich dementsprechend gegenseitig mehr oder weniger stören, können auf wenigstens eine andere Nährmittelkomponente verteilt werden.

Beispielsweise können in einer Nährstoffkomponente ein Carbonat und Ethyldiamintetraacetsäure vorhanden sein, insbesondere Natriumcarbonat. In einer anderen Nährstoffkomponente können ein Sulfat und Zitronensäure vorhanden sein, insbesondere Magnesiumsulfat.

In einer weiteren Ausführungsform wird vorgeschlagen, dass das Nährmedium ein Nährmedium für Mikroalgen (prokaryotische Cyanobakterien und eukaryotische Algen) ist, insbesondere das Nährmedium BG11.

In einer Fortbildung der Ausführungsform des letzten Absatzes wird zur Aufteilung der Nährstoffe des Nährmediums BG11 vorgeschlagen, dass eine erste Nährmittelkomponente Natriumnitrat und Calciumchlorid aufweist, eine zweite Nährmittelkomponente Natriumcarbonat und Natriumethyldiamintetraacetsäure aufweist und eine dritte Nährmittelkomponente Magnesiumsulfat, Eisenammoniumcitrat und Zitronensäure aufweist.

In einer weiteren Fortbildung wird vorgeschlagen, dass
- eine erste Nährmittelkomponente Natriumnitrat und Calciumchlorid aufweist, bevorzugt zwischen 50% und 70% Natriumnitrat, besonders bevorzugt zwischen 59% und 61%, sowie bevorzugt zwischen 0,5% und 2,5% Calziumchlorid, besonders bevorzugt zwischen 1,3% und 1,6%,
- eine zweite Nährmittelkomponente Natriumcarbonat, Dikaliumhydrogenphosphat und Natriumethyldiamintetraacetsäure aufweist, bevorzugt zwischen 3% und 8% Natriumcarbonat, besonders bevorzugt zwischen 5% und 6%, sowie bevorzugt zwischen 5% und 15% Dikaliumhydrogenphosphat, besonders bevorzugt zwischen 10% und 11%, sowie bevorzugt bis zu 1% Natriumethyldiamintetraacetsäure, besonders bevorzugt zwischen 0,2% und 0,4%,
- und eine dritte Nährmittelkomponente Magnesiumsulfat, Eisenammoniumcitrat und Zitronensäure aufweist, bevorzugt zwischen 10% und 20%, besonders bevorzugt zwischen 11% und 14%,
wobei sich die in diesem Anspruch vorstehend genannten Prozentangaben auf das Gesamtgewicht der jeweiligen portionierten Nährmittelkomponente beziehen, wobei das Gesamtgewicht der ersten Nährmittelkomponente zwischen 25,9% und 48,2% Füllstoffe, bevorzugt zwischen 36% und 40% Füllstoffe, aufweist, das Gesamtgewicht der zweiten Nährmittelkomponente zwischen 59,0% und 76,7%, bevorzugt zwischen 63% und 67% Füllstoffe aufweist, und das Gesamtgewicht der dritten Nährmittelkomponente zwischen 70% und 90%, bevorzugt zwischen 75% und 79% Füllstoffe aufweist.

Diese Kombination von Merkmalen ermöglicht eine akzeptable Haltbarkeit der Nährmittelkomponenten. D

Weiter wird ein Verfahren zum Herstellen eines Nährmediums für biologische Organismen oder Zellsysteme vorgeschlagen, wobei ein Nährmedium mittels eines Nährmitttelsystems gemäß einer der vorstehend beschriebenen Ausführungsform ansetzbar bzw. regenerierbar ist, indem einer Flüssigkeit, die ein Nährmedium bildet, ein Nährmittel hinzugefügt wird, das verschiedene Nährstoffe enthält.

In einer Weiterbildung des Verfahrens gemäß dem vorstehenden Absatz werden aus der Nährlösung vor dem Gebrauch unlösliche Komponenten wie etwa Füllstoffe, insbesondere von Tabletten, abfiltriert. Auf diese Weise fallen weniger dispergierte Schwebstoffe und weniger Schlamm in dem Bioreaktor an.

## Patentansprüche

1. Nährmittelsystem für biologische Organismen oder Zellsysteme, wobei mit dem Nährmitttelsystem ein Nährmedium ansetzbar bzw. regenerierbar ist, indem einer Flüssigkeit, die ein Nährmedium bilden soll, oder einem bestehenden Nährmedium ein verschiedene Nährstoffe enthaltendes Nährmittel hinzugefügt wird, wobei das Nährmittel miteinander unverträgliche Nährstoffe enthält, die die Haltbarkeit des Nährmittels im Lagerzustand verringern, wenn Sie miteinander reagieren, **dadurch gekennzeichnet, dass** das Nährmittelsystem wenigstens zwei Nährmittelkomponenten enthält, wobei wenigstens eine Nährmittelkomponente mehrere verschiedene miteinander verträgliche Nährstoffe enthält, und wobei zumindest zwei miteinander unverträgliche Nährstoffe in verschiedene Nährmittelkomponenten eingebracht sind.

2. Nährmittelsystem nach Anspruch 1, **dadurch gekennzeichnet, dass** Nährstoffe in dem Nährmittelsystem portioniert sind, wobei insbesondere eine oder mehrere Nährmittelkomponenten zu einer Tablette gepresst oder als kristalliner oder polykristalliner Block ausgebildet sind, wobei optional die Nährstoffe mehrerer Nährmittelkomponenten in verschiedenen Abschnitten einer Tablette oder des Blocks angeordnet sind, oder eine Nährmittelkomponente als eine Pulverportion verpackt ist, insbesondere in einer Kapsel, oder als eine definierte Menge von flüssiger Lösung, insbesondere als Konzentrat, in einem Behälter verpackt ist, beispielsweise in einer Gelkapsel.

3. Nährmittelsystem nach Anspruch 2, **dadurch gekennzeichnet, dass** Mengen der Portionen der Nährmittelkomponenten und Zusammensetzungen der Nährmittelkomponenten derart aufeinander abgestimmt sind, dass eine ganzzahlige Kombination von Portionen von Nährmittelkomponenten ein bestimmtes Nährmedium ergibt, insbesondere ein Standard-Nährmedium nach einem einschlägigen Rezept, beispielsweise das Nährmedium BG11.

4. Nährmittelsystem nach einem der Ansprüche 2 oder 3, **dadurch gekennzeichnet, dass** das Nährmittelsystem einen Basisnährstoff oder einen Satz von Basisnährstoffen aufweist, der bzw. die der Ernährung der erwünschten Organismen mit zumindest einem Grundnährstoff dienen, der bzw. die deren Überleben sichern, und optional einen oder mehrere Zusatznährstoffe aufweist, durch die eine besondere Beeinflussung der Organismen stattfindet, wie etwa verstärktes Wachstum oder die Herstellung bestimmter Ernährungszustände und/oder Anregung der Erzeugung bestimmter Stoffe im Stoffwechsel der Organismen, wobei die Zusatznährstoffe insbesondere in anderen Nährmittelkomponenten enthalten sind als die Nährmittelkomponenten, die Basisnährstoffe enthalten, wobei ein Zusatznährstoff bevorzugt in einer Nährmittelkomponente enthalten ist, die keine anderen Nährstoffe enthält.

5. Nährmittelsystem nach einem der vorangehenden Ansprüche, **dadurch gekennzeichnet, dass** ein mit dem Nährmittelsystem hergestelltes Nährmedium als Mangelmedium herstellbar oder regenerierbar ist, indem in den Nährstoffkomponenten eine hohe Reinheit der Nährstoffe vorgesehen ist.

6. Nährmittelsystem nach einem der vorangehenden Ansprüche, **dadurch gekennzeichnet, dass** die Portionierung wenigstens einer Nährmittelkomponente, die insbesondere wenigstens einen Nährstoff eines Zusatznährmittels enthält, derart klein gewählt wird, dass zumindest eine Konzentration wenigstens eines Nährstoffs in dem Nährmedium durch die Anzahl der Portionen dieser Nährmittelkomponenten einstellbar ist, insbesondere der Stickstoff-, Phosphor- und / oder Salzgehalt, wobei bevorzugt eine ganzzahlige Anzahl von Portionen eine Normalkonzentration des Stoffs oder der Stoffe in der Nährmittelkomponente ergibt und/oder wobei vorzugsweise für die Einstellung der Konzentration des oder der betreffenden Nährstoffe wenigstens zwei Stufen vorgesehen sind.

7. Nährmittelsystem nach einem der vorangehenden Ansprüche, **dadurch gekennzeichnet, dass** eine Nährmittelkomponente wenigstens zwei Salze umfasst, wobei die zu den Anionen der Salze gehörigen Säuren in ihrer Säurestärke nicht mehr als um einen akzeptablen Salz-Salz-Säurestärkefaktor voneinander abweichen, wobei zur Berechnung des Salz-Säurestärkefaktors die Säurestärke der stärkeren Säure durch die Säurestärke der schwächeren Säure geteilt wird.

8. Nährmittelsystem nach einem der vorangehenden Ansprüche, **dadurch gekennzeichnet, dass** eine Nährmittelkomponente wenigstens ein Salz und wenigstens eine Säure umfasst, wobei die Säurestärken der Säure und einer Säure, die zu dem Anion des Salzes gehört, um nicht um mehr als einen akzeptablen Säure-Salz-Säurestärkefaktor voneinander abweichen, wobei zur Berechnung des Säure-Salz-Säurestärkefaktors die Säurestärke der Säure durch die Säurestärke der Säure des Anions des Salzes geteilt wird.

9. Nährmittelsystem nach einem der vorangehenden Ansprüche, **dadurch gekennzeichnet, dass** Salze in dem Nährmittelsystem gemäß der Säurestärke ihrer Anionen auf verschiedene Nährmittelkomponenten aufgeteilt sind, wobei in wenigstens eine Nährmittelkomponente wenigstens zwei Salze mit einer Säurestärke, die weniger als um einen vorbestimmten Säurestärkefaktor voneinander abweichen, eingebracht sind.

10. Nährmittelsystem nach einem der vorangehenden Ansprüche, **dadurch gekennzeichnet, dass** das Nährmittelsystem wenigstens eine Nährmittelkomponente aufweist, welche ein Salz und wenigstens eine Säure mit einer geringeren Säurestärke als die Säurestärke der Säure des Anions des Salzes aufweist.

11. Nährmittelsystem nach einem der vorangehenden Ansprüche, **dadurch gekennzeichnet, dass** das Nährmedium ein Nährmedium für Mikroalgen und/oder Zellkulturen ist, insbesondere das Nährmedium BG11.

12. Nährmittelsystem nach Anspruch 11, **dadurch gekennzeichnet, dass**
• eine erste Nährmittelkomponente Natriumnitrat und Calciumchlorid aufweist, bevorzugt zwischen 50% und 70% Natriumnitrat, besonders bevorzugt zwischen 59% und 61%, sowie bevorzugt zwischen 0,5% und 2,5% Calciumchlorid, besonders bevorzugt zwischen 1,3% und 1,6%,
• eine zweite Nährmittelkomponente Natriumcarbonat, Dikaliumhydrogenphosphat und Natriumethyldiamintetraacetsäure aufweist, bevorzugt zwischen 3% und 8% Natriumcarbonat, besonders bevorzugt zwischen 5% und 6%, sowie bevorzugt zwischen 5% und 15% Dikaliumhydrogenphosphat, besonders bevorzugt zwischen 10% und 11%, sowie bevorzugt bis zu 1% Natriumethyldiamintetraacetsäure, besonders bevorzugt zwischen 0,2% und 0,4%,
• und eine dritte Nährmittelkomponente Magnesiumsulfat, Eisenammoniumcitrat und Zitronensäure aufweist, bevorzugt zwischen 10% und 20%, besonders bevorzugt zwischen 11% und 14%,
wobei sich die in diesem Anspruch vorstehend genannten Prozentangaben auf das Gesamtgewicht der jeweiligen portionierten Nährmittelkomponente beziehen, wobei das Gesamtgewicht der ersten Nährmittelkomponente zwischen 25,9% und 48,2% Füllstoffe, bevorzugt zwischen 36% und 40% Füllstoffe, umfasst, das Gesamtgewicht der zweiten Nährmittelkomponente zwischen 59,0% und 76,7%, bevorzugt zwischen 63% und 67% Füllstoffe umfasst, und das Gesamtgewicht der dritten Nährmittelkomponente zwischen 70% und 90%, bevorzugt zwischen 75% und 79% Füllstoffe umfasst.

13. Verfahren zum Herstellen eines Nährmediums für biologische Organismen oder Zellsysteme, wobei ein Nährmedium mittels eines Nährmitttelsystems gemäß einem der Ansprüche 1 bis 12 ansetzbar bzw. regenerierbar ist, indem einer Flüssigkeit, die ein Nährmedium bildet, ein Nährmittel hinzugefügt wird, das verschiedene Nährstoffe enthält.

14. Verfahren nach einem der vorangehenden Ansprüche, **dadurch gekennzeichnet, dass** aus der Nährlösung vor dem Gebrauch unlösliche Komponenten wie etwa Füllstoffe, insbesondere von Tabletten, abfiltriert werden, insbesondere mikrokristalline Zellulose.
